Europäisches Patentamt

European Patent Office

Office européen des brevets

(19)

(11) EP 0 539 660 B1

(12) **EUROPÄISCHE PATENTSCHRIFT**

(45) Veröffentlichungstag und Bekanntmachung des Hinweises auf die Patenterteilung:
**28.05.1997 Patentblatt 1997/22**

(51) Int Cl.$^6$: **C12N 13/00**, H01S 3/00

(21) Anmeldenummer: **92111456.7**

(22) Anmeldetag: **06.07.1992**

(54) **Mikromanipulatorisches Verfahren und Vorrichtung zur Anwendung bei der In Vitro Fertilisation**

Process of micromanipulation and apparatus with utilization for in vitro fertilization

Procédé de micromanipulation et appareil à utilisation en fertilisation in vitro

(84) Benannte Vertragsstaaten:
**AT DE FR GB**

(30) Priorität: **30.10.1991 DE 9113506 U**
**16.03.1992 DE 4208289**

(43) Veröffentlichungstag der Anmeldung:
**05.05.1993 Patentblatt 1993/18**

(73) Patentinhaber:
- **FUHRBERG TEICHMANN WINDOLPH LISA LASER PRODUCTS OHG**
  **37191 Katlenburg-Lindau (DE)**
- **Feichtinger, Wilfried, Dr.**
  **A-1130 Wien (AT)**

(72) Erfinder:
- **Fuhrberg, Peter, Dr.**
  **W-3400 Göttingen (DE)**

- **Feichtinger, Wilfried, Prof. Dr.**
  **A-1130 Wien (AT)**

(74) Vertreter: **Rehberg, Elmar, Dipl.-Ing.**
**Patentanwalt**
**Postfach 31 62**
**37021 Göttingen (DE)**

(56) Entgegenhaltungen:
**EP-A- 0 214 712      EP-A- 0 252 409**
**EP-A- 0 325 836      US-A- 4 988 163**

- **HEALTH PERIODICALS DATABASE, Forster City US, Ab.No. 11471455 Noble, Marcus 'Putting laser medical research...'**
- **PTS NEWSLETTER DATABASE, CLEVELAND, US Ab No. 01472638**

**Beschreibung**

Die Erfindung bezieht sich auf ein mikromanipulatorisches Verfahren zur Anwendung bei der In Vitro Fertilisation, bei dem die Zona pellucida einer Eizelle lokal mit einem Laserstrahl abgetragen wird, und eine Vorrichtung zur Durchführung des Verfahrens. Eine in immer stärkerem Umfang beobachtete Kulturkrankheit ist die relative Bewegungsinsuffizienz männlicher Samenzellen. Diese reicht soweit, daß die Samenzellen aus eigener Kraft nicht mehr in der Lage sind, die Zona pellucida einer Eizelle zur Befruchtung zu durchdringen. Eine Möglichkeit, das hieraus resultierende Befruchtungsversagen bei der In Vitro Fertilisation zu überwinden, besteht darin, die Zona pellucida gezielt, aber ohne weitergehende Beschädigung der Eizelle, zu schwächen, bzw. umschrieben zu durchbrechen. Weiterhin wird, nach erfolgter künstlicher Befruchtung vielfach beobachtet, daß das Einnisten der retransplantierten Embryonen in den Uterus scheitert, weil die Blastocyste nicht aus der sie umgebenden Zona pellucida zu schlüpfen vermag. Bekanntermaßen ist auch die Einnist-Wahrscheinlichkeit befruchteter Eizellen im Uterus durch gezieltes Schwächen der Zona pellucida ("Assisted Hatching") deutlich vergrößerbar.

Ein Verfahren und eine Vorrichtung der eingangs beschriebenen Art werden in der DE-Z Spektrum der Wissenschaft, Heft August 1991, auf Seite 92 beschrieben. Mit dem Laserstrahl eines ExcimeráLasers von 0,193 µm optischer Wellenlänge wurden Löcher durch die Zona pellucida einer Kanincheneizelle gebohrt. Auf diese Weise ließ sich die Befruchtungsrate steigern, da die Samenzellen nunmehr eine unmittelbare Zutrittsmöglichkeit in das Innere der Eizelle hatten. Gegen die Verwendung eines Excimer-Lasers zur Behandlung von desoxyribonukleinsäurehaltigen Zellen bestehen jedoch erhebliche Bedenken. Das Absorptionsmaximum von Desoxyribonukleinsäure liegt bei einer optischen Wellenlänge von 0,268 nm. Bereits dies läßt Schlüsse darauf zu, daß die Laserstrahlung eines Excimer-Lasers zu unkontrollierten Mutationen innerhalb der bestrahlten Zelle führt.

Es liegt aber auch eine unmittelbare Untersuchung der Mutagenität von mit den Laserstrahlen von Excimer-Lasern bestrahlten Eierstockzellen vor. Rasmussen et al. berichten in dem Artikel "Mutation and sister chromatid exchange induction in Chinese Hamster ovary (CHO) cells ..." (Gb-Z Photochem. Photobiol., Heft April 1989, Seiten 413 ff.) von außerordentlich hohen Mutationsraten bei Eierstockzellen des Chinesischen Hamsters, die zuvor mit Laserstrahlen von Excimer-Lasern bestrahlt worden waren. Die dabei verwendeten optischen Wellenlängen lagen bei 193 und 308 nm.

Bei einem tatsächlich praktizierten Stand der Technik wird die Zona pellucida einer Eizelle sowohl zum erleichterten Eindringen einer Samenzelle als auch zum erleichterten Einnisten in den Uterus mit einer Glaslanzette eingeritzt bzw. angekerbt. Hierbei wird die Eizelle durch Unterdruck an der Öffnung einer Haltepipette fixiert. Die mechanische Bearbeitung der Eizelle ist jedoch mit zwei Gefahren verbunden. Zum einen ist nicht auszuschließen, daß bei einem Durchtrennen der Zona pellucida auch eine Beschädigung des dahinterliegenden Ooplasmas erfolgt. Zum anderen wird die gesamte Eizelle bereits durch das Aufsetzen der Glaslanzette mechanisch beansprucht, woraus ebenfalls ungewollt eine Beschädigung des Zellinneren resultieren kann.

Als wesentlicher Nachteil dieser mechanischen Methode beim Erleichtern des Einnistens im Uterus ist aus dem Artikel "Implantation enhancement by selective assisted hatching using zona drilling of human embryos with poor prognosis" (Cohen et al., Z Hum. Reprod., 7. Jahrgang Nummer 5, Seiten 685 bis 691 (1992)) bekannt, daß die Größe der mit der Glaslanzette geschaffenen Öffnung nicht genau genug bestimmbar ist. In dem Fall eines zu kleinen Lochs besteht aber die Gefahr, daß das Schlüpfen der Blastocyste nicht vollständig erfolgt, woraufhin der Embryo zugrunde geht. Durch eine zu große Öffnung in der Zona pellucida kann andererseits die Blastocyste schon während des intrauterinen Embryotransfers verloren gehen, womit ein Einnisten von vorn herein unterbleibt.

Bei einem weiteren praktizierten Stand der Technik wird die Zona pellucida durch den Einsatz einer sauren Lösung geschwächt, d. h. in einem umschriebenen Bereich weggeätzt. Es versteht sich, daß hierbei die Kontrolle des Ätzvorgangs und damit das Verhindern von über das gewollte Maß hinausgehenden Schädigungen der Zona pellucida, bzw. des Ooplasmas schwer zu gewährleisten sind.

Zur Anwendung bei endoskopisch orthopädischen Eingriffen ist das "YACHT 2J-20 Holmium: YAG Laser System" der Firma "LISA laser products" bekannt. Hierbei wird der Laserstrahl eines Holmium-YAG-Lasers (ein Laser auf der Basis eines holmiumdotierten Yttrium-Aluminium-Granat-Kristalls) über einen Lichtleiter direkt auf die zu behandelnden Stelle des Operationsgebietes gerichtet. Der Laserstrahl des Holmium-YAG-Lasers weist eine optische Wellenlänge von 2,1 µm auf. Hieraus resultiert bei der Puls-Spitzen-Leistung des Holmium-YAG-Lasers von einigen kW eine effektive Reichweite in wässrigen Medien von ca. 300 µm. Im Vergleich dazu beträgt die Dicke der Zona pellucida einer Eizelle etwa 10 bis 20 µm.

Vorteilhaft verheilen jedoch mit dem Laserstrahl eines Holmium-YAG-Lasers ausgeführte Schnitte sehr rasch, was auf geringe Auswirkungen auf die Desoxyribonukleinsäuren der Zellen im Randbereich des Schnitts schließen läßt.

Es sind Erbium-YAG-Laser bekannt, die einen Laserstrahl mit einer optischen Wellenlänge von 2,94 µm emittieren. Der Erbium-YAG-Laser findet in der Medizintechnik bislang jedoch kaum Anwendung, da derzeit noch keine Lichtleiter bekannt sind, die einen Laserstrahl mit solcher Wellenlänge ohne übermäßige Dämpfung zu leiten vermögen und gleichzeitig den normalen mechanischen und thermischen Beanspruchungen standhalten. Fasern aus Zirkonfluorid transmittieren zwar Laserstrahlen mit einer Wellenlänge oberhalb 2,5 µm, sie zeichnen sich jedoch nachteiligerweise durch eine große Feuchtempfindlichkeit bei gleichzeitig hygroskopischen Eigenschaften, eine geringe Bruchfestigkeit sowie eine niedrige Zerstörungsschwelle bezogen auf die pro Querschnittsfläche transmittierte Lichtleistung aus. Zudem ist Zirkonfluorid toxisch.

In der Zahnmedizin finden Erbium-YAG-Laser in Verbindung mit Spiegelgelenkarmen zur Übertragung der Laserstrahlen Verwendung. Spiegelgelenkarme sind jedoch wegen ihres komplizierten Aufbaus unpraktisch zu handhaben und auch bereits wegen ihrer großen räumlichen Abmessungen für die Verwendung in einer Vielzahl medizinischer Einsatzbereiche nicht geeignet.

Der Erfindung liegt die Aufgabe zugrunde ein Verfahren und eine Vorrichtung aufzuzeigen, mit denen die Zona pellucida einer Eizelle gezielt und kontrolliert abgetragen werden kann, ohne daß eine Gefahr für die Eizelle oder für einen innerhalb der Zona pellucida durch Zellteilung entwickelten Embryo besteht.

Erfindungsgemäß wird dies bei einem Verfahren der eingangs beschriebenen Art dadurch gelöst, daß der Laserstrahl eine optischen Wellenlänge zwischen 1,2 und 8 µm aufweist. In dem Bereich von 1,2 bis 8 µm sind die Maxima des Absorptionsspektrums von Wasser zu finden. Hieraus ergeben sich verhältnismäßig geringe Reichweiten eines entsprechenden Laserstrahls in wässrigen Medien. Als solches ist auch die Zona pellucida einer Eizelle anzusehen. Bei einer angemessen geringen Ausgangsleistung des Laserstrahls bleibt die effektive Reichweite sogar kleiner als die Dicke der Zona pellucida. Dies gestattet wiederum eine Bearbeitung der Zona pellucida, ohne daß der Laserstrahl das Innere der Eizelle erreicht und dort zu Zerstörungen oder Mutationen führen könnte.

In dem Bereich zwischen 1,2 und 8 µm weist das Absorptionsspektrum von Wasser insgesamt 5 Maxima auf. Jedes dieser Maxima bezeichnet optische Wellenlängen, mit denen ein entsprechender Laserstrahl besonders gut für das neue Verfahren geeignet ist. Das höchste Maximum liegt bei ca. 3 µm optischer Wellenlänge. Hieraus ergibt sich für den entsprechenden Laserstrahl die geringste relative Reichweite in wässrigen Medien auf, so daß er besonders vorteilhaft zur kontrollierten Bearbeitung der Zona pellucida einsetzbar ist.

Der Laserstrahl kann einen Durchmesser von 3 bis 30 µm aufweisen. Im wässerigen Medien sind mit einem solchen Laserstrahl die zur Bearbeitung der Zona pellucida notwendige Leistungsdichten möglich, ohne daß eine unerwünscht hohe effektive Reichweite des Laserstrahls über die Dicke der Zona pellucida hinaus auftritt.

Der Laserstrahl kann gepulst sein, wobei jeder Puls eine Energie von ca. 10 - 100 µJ aufweist. Ein gepulster Laserstrahl bietet sich deshalb an, weil so einerseits die zur Bearbeitung der Zona pellucida notwendigen Puls-Spitzen-Leistung zur Verfügung steht, aber andererseits eine unerwünschte Aufheizung des bearbeiteten Bereichs vermieden wird. Als besonders günstig hat sich eine Energie pro Puls von ca. 10 - 100 µJ erwiesen, wobei ca. 5 bis 10 Pulse notwendig sind, um ein durchgehendes Loch in die Zona pellucida einzubringen.

Die Zona pellucida kann mit dem Laserstrahl zum erleichterten Eindringen einer Samenzelle lokal durchbrochen werden. Aber auch eine Schwächung der Zona pellucida kann bei nur teilweise in ihrer Mobilität geschwächten Samenzellen ausreichen, um die Befruchtung der Eizelle zu ermöglichen.

Darüberhinaus ist das Einbringen einer umschriebenen Öffnung oder eine Schwächung der Zona pellucida mit dem Laserstrahl sinnvoll, bevor die zuvor befruchtete und bereits zu einem mehrzelligen Embryo weiterentwickelte Eizelle in den Uterus der jeweiligen Patientin überführt wird. Dies unter anderem deshalb, weil Hinweise auf die Härtung der Zona pellucida durch die in vitro Kultivierung der Eizellen vorliegen, bzw.an manchen Eizellen die Zona unphysiologisch stark ausgebildet ist. Hierdurch wird die Wahrscheinlichkeit des Einnistens der Eizelle im Uterus um ein Vielfaches gesteigert, während eine unbehandelte Zona pellucida das Schlüpfen der Eizelle erschwert.

Bei einer Vorrichtung zur Durchführung des Verfahrens wird die Aufgabe erfindungsgemäß durch die Merkmale des Anspruchs 7 gelöst. Erbium-dotierte Festkörperlaser weisen optische Wellenlängen im Bereich des Hauptmaximums des Wasserabsorptionsspektrums auf.

Die besonders vorteilhafte Wellenlänge von 3 µm wird insbesondere mit einem Erbium-YAG-Laser gut erreicht. Dieser Lasertyp weist eine optische Wellenlänge von 2,944 µm auf. Die optische Wellenlänge eines Erbium-YSGG-Lasers von 2,796 µm fällt hingegen schon in die Flanke des Hauptmaximums. In Bereiche anderer Absorptionsmaxima des Wasserabsorptionsspektrums fallen die Laserstrahlen von Holmium-YAG-Lasern mit 2,12 µm und von Thulium-YAG-Lasern mit 1,9 bis 2,0 µm.

Der Lichtleiter ist eine sich zwischen ihrem proximalen Ende zu ihrem distalen Ende verjüngende, wasserarme oder wasserfreie Quarz-Quarz-Faser. Für die neue Vorrichtung ist es weitgehend unschädlich, daß für Laserstrahlen über einer gewissen optischen Wellenlänge keine strapazierbaren Lichtleiter mit großen Transmissionskoeffizienten zur Verfügung stehen. Ganz im Gegenteil wird die hohe Dämpfung weitgehend herkömmlicher Lichtleiter sogar gezielt ausgenutzt . Laser sind nämlich nur deutlich oberhalb ihrer Laser-Schwell-Energie präzise ansteuerbar. Die hier anfallenden Energien gehen jedoch weit über das Maß hinaus, das zur Bearbeitung der Zona pellucida einer Eizelle notwendig und geeignet ist. Der Laserstrahl eines bei verhältnismäßig hoher Leistung exakt geregelten Lasers wird daher bei der Erfindung durch einen Lichtleiter mit hohem Absorptionskoeffizienten auf das sinnvolle Maß herabgedämpft. Es ist allerdings darauf zu achten, daß der verwendete Lichtleiter möglichst wasserarm oder gar wasserfrei ist, um in dem in Rede stehenden optischen Wellenlängenbereich wenigstens die unbedingt notwendige Mindesttransmissionsfähigkeit aufzuweisen. Mit der sich verjüngenden Ausbildung der Quarz-Quarz-Faser ist ein notwendiger Konzentrationseffekt des eingespeisten Laserstrahls auf die Bearbeitungsstelle verbunden.

Die Quarz-Quarz-Faser weist an ihrem distalen Ende einen Kerndurchmesser von 3 bis 30 µm auf, wobei der Kerndurchmesser an ihrem proximalen Ende etwa 10 mal größer ist. Diese Ausbildung des Lichtleiters erlaubt ein kontrolliertes Einspeisen des Laserstrahls und ist mit einer auf den Anwendungszweck abgestimmten Konzentrationswirkung verbunden.

Die Quarz-Quarz-Faser weist unter Zugrundelegung eines logarithmischen Abschwächungsgesetzes:

$$EXP -(Absorptionskoeffizient * Faserlänge)$$

einen Absorptionskoeffizienten von etwa 0,2 bis 0,4 1/cm für den Laserstrahl des Lasers auf. Bei diesem Absorptionskoeffizienten und einer praktischen Faserlänge von etwa 0,5 m ergibt sich ein Dämpfungsfaktor zwischen $10^{-3}$ und $10^{-9}$, bei dem die für die gewünschte Ausgangspulsenergie am distalen Ende der Quarz-Quarz-Faser notwendige Eingangspulsenergie des Lasers gut zu regeln ist.

Die Erfindung wird im Folgenden anhand von Ausführungsbeispielen näher erläutert und beschrieben. Es zeigt:

Figur 1    die Vorrichtung zur Durchführung des mikromanipulatorischen Verfahrens zur Anwendung bei der In Vitro Fertilisation,

Figur 2    ein Wasserabsorptionsspektrum,

Figur 3    eine Laserkennlinie zu der Vorrichtung gemäß Figur 1 und

Figur 4    die Durchführung des mikromanipulatorischen Verfahrens bei der In Vitro Fertilisation.

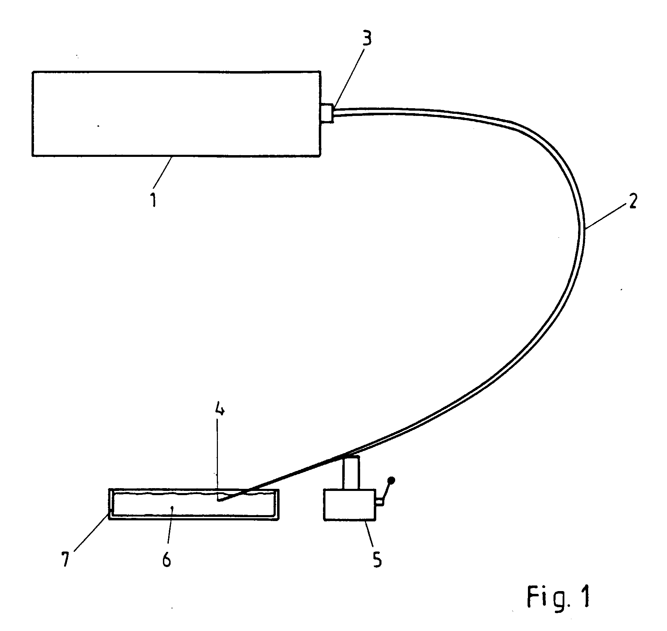

Die in Figur 1 dargestellte Vorrichtung besteht im wesentlichen aus einem Erbium-YAG-Laser 1 und einem mit diesem verbundenen Lichtleiter 2. Der Erbium-YAG-Laser 1 gibt einen hier nicht dargestellten Laserstrahl mit einer optischen Wellenlänge von 2,94 µm ab. Dieser wird in den Lichtleiter 2 eingekoppelt. Der Lichtleiter ist eine wasserfreie Quarz-Quarz-Faser. Eine für den Lichtleiter geeignete Faser wird beispielsweise von der Firma "CeramOptec" unter der Bezeichnung "WF 100/120A" vertrieben. Der Lichtleiter 2 weist für den Laserstrahl des Lasers 1 einen Absorptionskoeffizienten von etwa 0,2 bis 0,4 1/cm auf. Dies entspricht bei einer praktikablen Faserlänge zwischen 0,3 m und 0,5 m einem Dämpfungsfaktor zwischen $10^{-3}$ und $10^{-9}$. Der genaue Wert des Dämpfungsfaktors des Lichtleiters 1 ist jedoch chargenabhängig. Der Durchmesser des Lichtleiters 1 nimmt zwischen seinem proximalen Ende 3 und seinem distalen Ende 4 hin ab. Die Verjüngung erstreckt sich dabei aus fertigungstechnischen Gründen im wesentlichen in dem Bereich des Lichtleiters nahe seinem distalen Ende 4. Der Kerndurchmesser der Quarz-Quarz-Faser beträgt hierbei an dem distalen Ende 4 ca. 10 µm. An dem proximalen Ende 3 ist der Kerndurchmesser etwa 10 mal größer, d. h. er beträgt ca. 100 µm. Mit der konischen Ausbildung des Lichtleiters 2 sind verschiedene Vorteile verbunden. Zum einen erleichtert der größere Kerndurchmesser an dem proximalen Ende 3 die Einkopplung des Laserstrahls des Lasers 1. Zum anderen wird der Laserstrahl am distalen Ende 4 des Lichtleiters 2 auf einen geringen Durchmesser konzentriert. Hierbei ergibt aus der dem vergleichsweise hohen Dämpfungsfaktors des Lichtleiters, daß die aus dem Kerndurchmesser des Lichtleiters 1 resultierende Querschnittsfläche etwa in dem gleichen Maße abnimmt wie die verbleibende Lichtleistung des Laserstrahls. Für den Lichtleiter 2 ist ein Mikromanipulator 5 vorgesehen, mit dessen Hilfe sich das distale Ende 4 des Lichtleiters 2 kontrolliert verfahren läßt. Beim Einsatz taucht das distale Ende 4 des Lichtleiters 2 in eine mit einer Blutisotonischen wässerigen Salzlösung 6 (Eizellkulturmedium) gefüllte Petrischale (bzw. in die gefüllte Vertiefung eines Objektträger) 7 ein. In der wässerigen Salzlösung 6 weist der aus dem distalen Ende 4 des Lichtleiters 2 austretende Laserstrahl nur eine geringe effektive Reichweite von etwa 3 µm auf. Dies ist neben der geringen Lichtleistung, die von dem Lichtleiter 2 transmittiert wird, vor allem auf den hohen Absorptionskoeffizienten α von Wasser bei der optischen Wellenlänge des Erbium-YAG-Lasers 1 zurückzuführen.

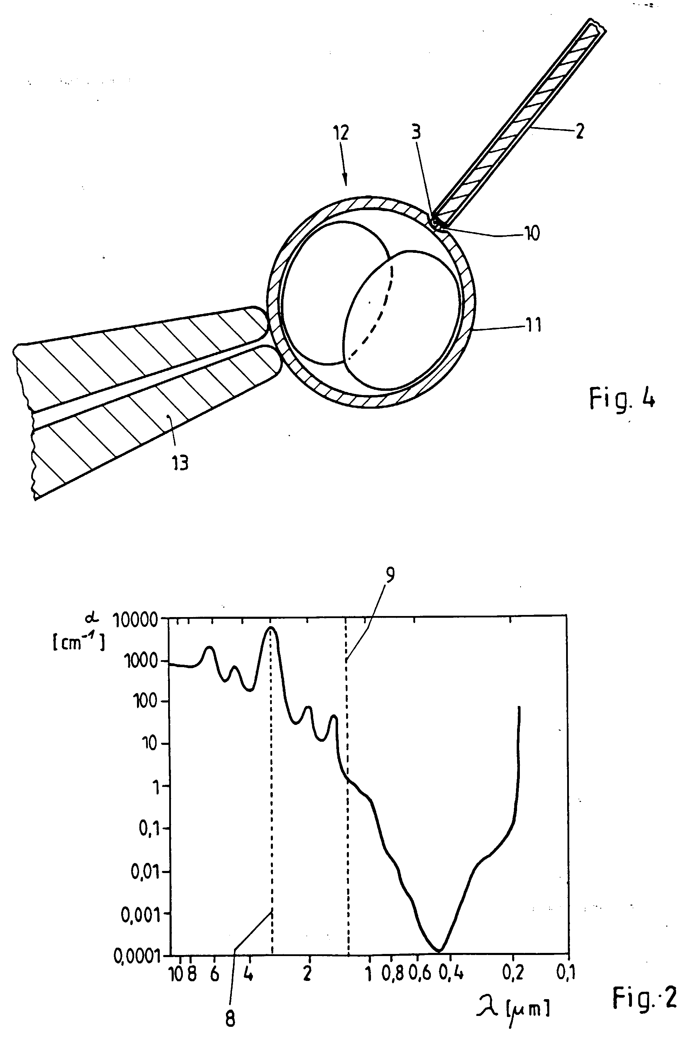

Das in Figur 2 dargestellte Absorptionsspektrum von Wasser zeigt den Absorptionskoeffizienten α aufgetragen über der optischen Wellenlänge. In dem Bereich der optischen Wellenlänge zwischen 1,2 und 8 µm weist der Absorptionskoeffizient α insgesamt fünf Maxima auf. Im Bereich dieser Maxima ist die Reichweite entsprechender Laserstrahlen in wässrigen Medien besonders klein. Die geringste Reichweite ergibt sich bei optischen Wellenlängen von ca. 3 µm. Dies entspricht nahezu exakt der Wellenlänge 8 des Erbium-YAG-Lasers 1. Demgegenüber resultiert die Wellenlänge 9 von 1,064 µm eines Neodymium-YAG-Lasers in deutlich größere Reichweiten. Die Wellenlänge 9 ist bereits in dem Randbereich des Minimums angeordnet, welches der Absorptionskoeffizient α bei ca. 0,5 µm optischer Wellenlänge ausbildet. Die hier nicht eingetragene Wellenlänge von 2,796 µm eines Erbium-YSGG-Lasers liegt hingegen noch im Bereich des höchsten Maximums des Absorptionskoeffizienten α.

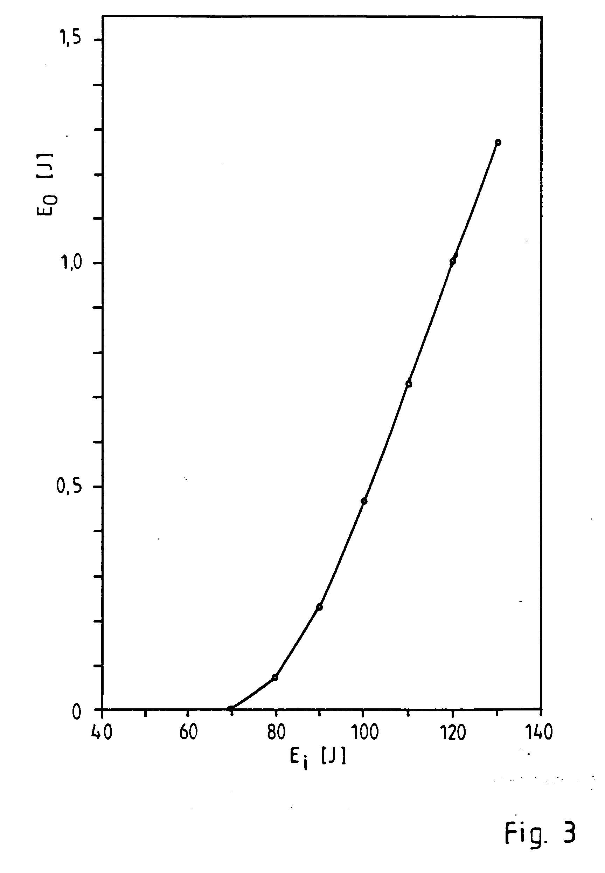

Das in Figur 3 wiedergegebene Diagramm stellt die von dem Erbium-YAG-Laser 1 abgestrahlte Energie $E_o$ in Abhängigkeit von der eingespeisten Pumpenergie $E_i$ dar. Eine Gegenüberstellung der mittleren Leistungen ist demgegenüber wenig sinnvoll, da diese von der jeweils gewählten Wiederholrate der Erbium-YAG-Laser 1 abhängig ist. Aus der Darstellung ist zu erkennen, daß geringe abgestrahlte Energien $E_o$ von einigen μJ pro Puls in den Bereich fallen, in denen der Erbium-YAG-Laser 1 erst beginnt, Strahlung abzugeben. Hier ist eine kontrollierte Steuerung der abgestrahlten Energie nicht möglich. Diese läßt sich hingegen in dem Bereich von 0,5 bis 1 Joule abgestrahlter Energie hervorragend realisieren. Damit ergeben sich jedoch andererseits Größenordnungen, die für die Bearbeitung von Eizellen nicht in Frage kommen. Bei der in Figur 1 dargestellten Vorrichtung wird der Ausgleich durch den hohen Dämpfungsfaktor des Lichtleiters 2 geschaffen.

Figur 4 zeigt die Durchführung des mikromanipulatorischen Verfahrens bei der In Vitro Fertilisation. Mit dem hier dargestellten Laserstrahl 10 wird die Zona pellucida 11 einer Eizelle 12 lokal abgetragen. Hierbei ist unter der Eizelle 12 eine unbefruchtete Eizelle, aber auch ein von der Zona pellucida 11 umschlossener Embryo nach ersten Zellteilungen zu verstehen. Die Eizelle 12 ist an einer Haltepipette 13 durch Unterdruck fixiert. Der Laserstrahl 10 wird durch Heranfahren des distalen Endes 4 an der Zona pellucida 11 angesetzt. Danach wird mit jedem Puls des Laserstrahls 10 die Zona pellucida 11 direkt vor dem distalen Ende 4 abgetragen. Die Stärke des Abtrags entspricht hierbei in etwa der Reichweite des Laserstrahls 10. Daher ist das distale Ende 4 nach jedem Puls weiter vorzufahren, bis es erneut auf der Zona pellucida 11 aufliegt. Für ein Durchbohren der Zona pellucida, deren Stärke typischerweise zwischen 10 und 20 μm beträgt, werden etwa 5 bis 10 Pulse des Laserstrahls 10 benötigt. Bei der Bearbeitung der Eizelle 12 mit dem Laserstrahl bleibt das innerhalb der Zona pellucida 11 angeordnete Zellinnere völlig unbeeinflußt. Der Laserstrahl 10 erreicht es aufgrund seiner geringen Reichweite nicht. Das distale Ende 4 des Lichtleiters 2 wird auf die Zona pellucida nur aufgesetzt und führt daher zu keinen mechanischen Belastungen der Eizelle. Damit sind wichtige Voraussetzungen erfüllt, die mitentscheidend dafür sind, daß die In Vitro Fertilisation mit dem neuen Verfahren eine auffällig hohe Erfolgsquote aufweist.

Eingesetzt werden kann das neue Verfahren grundsätzlich bei zwei verschiedenen Schritten der In Vitro Fertilisation. Zum einen kann eine Schwächung oder ein Durchbohren der Zona pellucida einer unbefruchteten Eizelle vorgenommen werden, um Samenzellen den Eintritt in das Innere der Eizelle zu erleichtern. Hierbei ist es darüberhinaus möglich, einzelne Samenzellen mit einer durch den Laserstrahl 10 vor dem distalen Ende 4 des Lichtleiters 2 optisch induzierten Druckwelle gezielt in die Eizelle einzuspülen. Andererseits kann die Zona pellucida einer befruchteten Eizelle, in der bereits erste Zellteilungen zur Ausbildung eines Embryos erfolgten, vor dem intrauterinen Embryotransfer geschwächt werden, um die Einnistung des Embryos im Uterus zu erleichtern.

## BEZUGSZEICHENLISTE

1 - Erbium-YAG-Laser
2 - Lichtleiter
3 - proximales Ende
4 - distales Ende
5 - Mikromanipulator
6 - Blut-isotonische Salzlösung
7 - Petrischale
8 - Wellenlänge Erbium-YAG-Laser
9 - Wellenlänge Neodymium-YAG-Laser
10 - Laserstrahl
11 - Zona pellucida
12 - Eizelle
13 - Haltepipette
14 - Zellen

## Patentansprüche

1. Mikromanipulatorisches Verfahren zur Anwendung bei der In Vitro Fertilisation, bei dem die Zona pellucida einer Eizelle lokal mit einem Laserstrahl abgetragen wird, dadurch gekennzeichnet, daß der Laserstrahl (10) eine optische Wellenlänge zwischen 1,2 und 8 μm aufweist.

2. Verfahren nach Anspruch 1, dadurch gekennzeichnet, daß der Laserstrahl (10) eine optische Wellenlänge von ca. 3 μm aufweist.

3. Verfahren nach Anspruch 1 oder 2, dadurch gekennzeichnet, daß der Laserstrahl (10) einen Durchmesser von 3 bis 30 μm aufweist.

4. Verfahren nach Anspruch 1, 2 oder 3, dadurch gekennzeichnet, daß der Laserstrahl (10) gepulst ist, wobei jeder Puls eine Energie von ca. 10 - 100 μJ aufweist.

5. Verfahren nach einem der Ansprüche 1 bis 4, dadurch gekennzeichnet, daß die Zona pellucida (12) mit dem Laserstrahl (10) zum erleichterten Eindringen einer Samenzelle lokal durchbrochen wird.

6. Verfahren nach einem der Ansprüche 1 bis 4, dadurch gekennzeichnet, daß die Zona pellucida (12) einer befruchteten Eizelle mit dem Laserstrahl (10) zum erleichterten Einnisten in den Uterus lokal geschwächt, bzw. durchbrochen wird.

7. Vorrichtung zur Durchführung des Verfahrens nach einem der voranstehenden Ansprüche 1 bis 6, mit einem Erbium-dotierten Festkörperlaser und einer wasserarmen oder wasserfreien Quarz-Quarz-Faser als Lichtleiter (1) für den Laserstrahl (10) des Lasers, **dadurch gekennzeichnet**, daß sich die Quarz-Quarz-Faser zwischen ihrem proximalen Ende (3) und ihrem distalen Ende (4) verjüngt, wobei sie an ihrem distalen Ende einen Kerndurchmesser von 3 bis 30 μm ausweist und daß die Quarz-Quarz-Faser für den Laserstrahl (10) des Lasers einen Dämpfungsfaktor zwischen $10^{-3}$ und $10^{-9}$ aufweist.

8. Vorrichtung nach Anspruch 7, **dadurch gekennzeichnet**, daß der Laser ein Erbium-YAG-Laser (1) oder ein Erbium-YSGG-Laser ist.

## Claims

1. Process of micromanipulation with utilization for in vitro fertilization, comprising the step of locally ablating the zona pellucida of an oocyte with a laser beam, characterized in that the laser beam (10) has an optical wavelength between 1.2 and 8 μm.

2. Process according to claim 1, characterized in that the laser beam (10) has an optical wavelength of approximately 3 μm.

3. Process according to claim 1 or 2, characterized in that said laser beam (10) has a diameter from 3 to 30 μm.

4. Process according to claim 1, 2 or 3, characterized in that the laser beam (10) is pulsed, each pulse having an energy approximately from 10 to 100 μJ.

5. Process according to any of the claims 1 to 4, characterized in that the zona pellucida (12) is locally opened with the said laser (10) for facilitating the entry of a sperm cell.

6. Process according to any of the claims 1 to 4, characterized in that the zona pellucida (12) of a fertilized oocyte is locally weakened or opened with said laser beam (10) for facilitating the lodging in an uterus.

7. Apparatus for carrying out the process according to any preceding claim 1 to 6, having an erbium doped solid state laser and a low-water content or water-free quartz-quartz fibre as a light guide (1) for the laser beam (10) of the laser, characterized in that the light guide tapers between its proximal end (3) and its distal end (4), having a core diameter from 3 to 30 μm at its distal end, and that the quartz-quartz fibre has an attenuation factor for the laser beam (10) of the laser between $10^{-3}$ and $10^{-9}$.

8. A device according to claim 7, characterized in that the laser is an erbium-YAG laser (1) or an erbium-YSSG laser.

## Revendications

1. Procédé de micromanipulation destiné à être utilisé dans la fertilisation in vitro, dans lequel la zona pellucida d'un ovule est enlevée localement avec un rayon laser, caractérisé en ce que le rayon laser (10) présente une longueur d'onde optique comprise entre 1,2 et 8 μm.

2. Procédé selon la revendication 1, caractérisé en ce que le rayon laser (10) présente une longueur d'onde optique d'environ 3 μm.

3. Procédé selon la revendication 1 ou 2, caractérisé en ce que le rayon laser (10) présente un diamètre compris entre 3 et 30 μm.

4. Procédé selon la revendication 1, 2 ou 3, caractérisé en ce que le rayon laser (10) est pulsé, chaque impulsion présentant une énergie comprise entre 10 et 100 μJ environ.

5. Procédé selon l'une des revendications 1 à 4, caractérisé en ce que la zona pellucida (12) est localement transpercée pour faciliter la pénétration d'un spermatozoïde.

6. Procédé selon l'une des revendications 1 à 4, caractérisé en ce que la zona pellucida (12) d'un ovule fécondé est localement affaiblie ou transpercée avec le rayon laser pour faciliter l'implantation dans l'utérus.

7. Dispositif pour la mise en oeuvre du procédé selon l'une des revendications 1 à 6 précédentes, comportant un laser à corps fixe dopé à l'erbium et une fibre quartz-quartz contenant peu d'eau ou exempte d'eau en tant que guide de lumière (1) pour le rayon laser (10) du laser, caractérisé en ce que la fibre quartz-quartz est rétrécie entre son extrémité proximale (3) et son extrémité distale (4), à son extrémité distale le diamètre du noyau étant compris entre 3 et 30 μm et en ce que la fibre quartz-quartz pour le rayon laser (10) du laser présente un facteur d'amortissement compris entre $10^{-3}$ et $10^{-9}$.

8. Procédé selon la revendication 7, caractérisé en ce que le laser est un laser YAG à erbium (1) ou un laser YSGG à erbium.

Fig. 1

Fig. 4

Fig. 2

Fig. 3